(19) Europäisches Patentamt
European Patent Office
Office européen des brevets

(11) Numéro de publication : **0 652 286 A1**

# (12) DEMANDE DE BREVET EUROPEEN

(21) Numéro de dépôt : **94420306.6**

(22) Date de dépôt : **09.11.94**

(51) Int. Cl.[6] : **C12N 15/82,** C12N 15/29, C12N 5/10, A01H 5/00

(30) Priorité : **10.11.93 FR 9313684**

(43) Date de publication de la demande : **10.05.95 Bulletin 95/19**

(84) Etats contractants désignés : **AT BE CH DE DK ES FR GB GR IE IT LI LU NL PT SE**

(71) Demandeur : **RHONE-POULENC AGROCHIMIE**
**14-20, rue Pierre Baizet**
**F-69009 Lyon (FR)**

(72) Inventeur : **Capellades, Montserrat**
**C/Sors 43, 2o-2a**
**E-08024 Barcelona (ES)**
Inventeur : **De Rose, Richard**
**216 Rue de Saint-Cyr**
**F-69009 Lyon (FR)**
Inventeur : **Montoliu, Lluis**
**Berliner Strasse 40, 6 OG**
**D-69120 Heidelberg (DE)**
Inventeur : **Puigdomenech, Pedro**
**C/Mare de Deu de Nuriq 24, 4oB**
**E-08017 Barcelone (ES)**
Inventeur : **Torres, Miguel Angel**
**Passeig Valldaura 243,**
**Principal 2a**
**E-08016 Barcelone (ES)**
Inventeur : **Rigau, Juan**
**C/Mareselva 6,**
**Sant Quirze del Vallés**
**E-08192 Barcelone (ES)**
Inventeur : **Uribe, Javier**
**C/Entença 162,**
**Principal 4a**
**E-08029 Barcelone (ES)**

(74) Mandataire : **Chrétien, François et al**
**RHONE-POULENC AGROCHIMIE- DPI**
**B.P. 9163**
**F-69263 Lyon Cédex 09 (FR)**

(54) **Eléments promoteurs de gènes chimères de tubuline alpha.**

(57)
1. Promoteur de plante
2. Il est issu d'un gène de la tubuline $\alpha$ du maïs
3. Utilisation pour la transformation génique des plantes.

EP 0 652 286 A1

Les microtubules sont des éléments essentiels pour de nombreuses fonctions dans toutes sortes de cellules. En particulier dans les plantes, ils jouent un rôle central dans plusieurs phénomènes importants, en particulier ceux relatifs à la morphogénèse. Dans tous les eucaryotes, les microtubules sont composés de bon nombre de protéines hautement conservées, les plus abondantes étant les tubulines. Deux sous unités principales de protéines ont été décrites, appelées tubuline α et tubuline β. Les fonctions essentielles des microtubules et leur distribution omniprésente ont souventsuggéré l'idée que les gènes codant pour les tubulines sont un bon exemple de gènes fortement et structurellement exprimés. En fait les sous unités de tubuline sont codées dans les eucaryotes par une famille de gènes. Dans le domaine végétal, les tubulines α et β ont été étudiées dans quelques plantes telles que le maïs, *Arabidopsis*, le soja, le pois, et la carotte. Dans tous ces cas, les génomes respectifs codent pour de multiples gènes de tubulines α et β, qui sont différemment exprimés durant le développement du végétal. Dans *Arabidopsis*, une analyse soigneuse d'une famille des gènes de tubulines a révélé 15 gènes (6 tubulines α et 9 tubulines β) codant pour ces protéines(Kopczak *et al.*, 1992; Snustad *et al.*, 1992). Dans le maïs, 3 gènes de tubulines α ont été clonés et séquencés (Montoliu *et al.*, 1989; Montoliu *et al.*, 1990), et six autres peuvent être détectés par analyse PCR de l'ADN génomique (Montoliu *et al.*, 1992). Selon une récente étude, au moins six séquences d'ADN de tubulines α distinctes ont été clonées et caractérisées à partir de tissus de maïs( Villemur *et al.*, 1992).

Les gènes *Tub* α *1* et *Tub* α *2*, issus du maïs sont arrangés en tandem, séparé par au moins 2 kilo paires de bases(kb) d'ADN. Ils sont exprimés dans tous les tissus méristématiques du maïs avec des niveaux élevés d'expression dans le système radiculaire (Montoliu *et al.*, 1989). Le gène *Tub* α *1* est exprimé dans tous les tissus analysés à un niveau plus élevé que le gène *Tub* α *2* et est en outre fortement exprimé dans le pollen (Montoliu *et al.*, 1990). Des expériences d'hybridation in vitro montrent qu'à l'intérieur des tissus méristématiques, le gène *Tub* α *1* est exprimé au cours de l'activation centrale quiescente (Rigau *et al.* in Press). Dans *Arabidopsis*, un gène s'est révélé spécifiquement exprimé dans le pollen mais aucun n'a montré le même patron d'expression que les gènes de maïs *Tub* α *1* ou *Tub* α *2* (Carpenter *et al.*, 1990). Le gène de maïs *Tub* α *3* est exprimé dans tous les organes végétaux qui sont riches en cellules en division, en particulier dans les embryons immatures (Montoliu *et al.*, 1990).

Il a maintenant été trouvé que les éléments de régulation promotrice des régions issues de gènes de tubuline α de maïs peuvent commander une expression tissulaire spécifique dans le pollen, les systèmes radiculaires, les zones méristématiques et les embryons immatures des espèces végétales tant monocotylédones que dicotylédones. La présente invention permet un plus grand contrôle de l'expression génique dans les plantes transgéniques, permettant notamment un meilleur contrôle des gènes de résistance herbicide.

La présente invention a pour objet la région régulatrice 5' d'un gène de tubuline α de maïs. Cette région est définie, dans la présente invention, comme un ensemble de régulation amont (ERA; en anglais upstream regulatory ensemble: URE), utile pour commander l'expression de gènes codant pour des protéines hétérologues. L'ERA comprend plusieurs éléments de régulation, qui créent des patrons d'expression régulée distincts, lorsqu'ils sont liés aux régions codantes de gènes hétérologues exprimés dans les plantes transgéniques. En particulier la présente invention fournit de l'information concernant les régions isolées de l'ADN issu de gènes de maïs *Tub* α *1*, qui commande spécifiquement l'expression des gènes dans les racines, le pollen et les tissus méristématiques.

Un autre aspect de l'invention concerne des gènes chimères de plantes contenant ces éléments de régulation. Les éléments de régulation sont liés de manière fonctionnelle à la séquence codante d'un gène hétérologue, de sorte que l'élément de régulation est capable de contrôler l'expression du produit codé par le gène hétérologue. Si nécessaire, d'autres éléments promoteurs, en totalité ou en partie, sont inclus dans les constructions de gènes chimères. Ces éléments complémentaires comprennent, mais ne sont pas limités, aux séquences d'introns de monocots tels que l'intron 1 du gène d'actine du riz, qui renforce beaucoup l'expression des gènes hétérologues dans les tissus de monocots. L'invention comprend aussi des vecteurs de transformation de plantes ainsi que les cellules végétales transformées par ces vecteurs et des plantes et les semences contenant le gène chimère.

Selon un autre aspect de l'invention, on produit des gènes chimères comprenant une séquence d'ADN codant pour un polypeptide de fusion contenant un transit peptide amino-terminal, qui est capable de diriger la localisation subcellulaire de peptides hétérologues vers les organelles subcellulaires spécifiques de cellules végétales, avec obtention d'un contrôle accru et un meilleur ciblage de l'expression génique dans les plantes transgéniques.

L'invention a également pour objet un procédé pour produire des plantes monocot ou dicot transformées, ayant une nouvelle propriété et notamment résistantes aux herbicides. Les cellules végétales transformées avec cette construction peuvent donner naissance, par régénération et/ou culture, à des plantes résistantes.

La présente invention comprend des éléments de régulation cis de l'ensemble de régulation amont (ERA) de gènes de tubuline α de maïs. Ces éléments de régulation cis sont des régions discrètes de l'ERA, qui confèrent une régulation de l'expression sur les gènes sous leur controle. En particulier, l'invention comprend un acide nucléique isolé contenant au moins un élément génique permettant une expression spécifique dans les racines, dans le pollen, dans les méristèmes et les embryons immatures.Tout gène de tubuline peut fournir les éléments de régulation, notamment , seuls ou en combinaison, les gènes de maïs *Tub* α *1*, *Tub* α *2* et *Tub* α *3*, qui représentent trois gènes tubuline α similaires. De préférence, on utilise le gène de tubuline α de maïs *Tub* α *1*, comme source d' éléments de régulation.

Un des éléments de régulation selon l'invention commande l'expression génique spécifique dans les racines. Un élément de régulation spécifique des racines comprend une séquence de nucléotide particulière, qui est capable de déclencher l'expression d'un gène sous son contrôle dans les racines, c'est à dire pour le produit d'expression du gène détecté dans les racines. L'expression qui est spécifique des racines peut se trouver dans toute partie de racine, par exemple mais de manière non limitative, les zones méristématiques de racines, les boutons latéraux de racines, les racines latérales et les zones vasculaires des racines. Aucune expression génique n'est decelée dans les pointes de pousses, les tiges ou les feuilles.

Pour identifier les éléments de régulation, qui commandent l'expression génique spécifique des racines, il faut effectuer une analyse par délétion de l'ERA entier d'un gène de tubuline α de maïs. Dans une analyse par délétion, on enlève successivement des nucleotides de l'ERA entier et les fragments résultants sont ligaturés à la séquence codante d'un gène rapporteur ou à une autre séquence codante hétérologue. Les constructions sont ensuite analysées pour leur aptitude à commander l'expression génique spécifiques des tissus en décelant la présence du produit du gène hétérologue dans les tissus spécifiques recherchés à l'exclusion d'autres tissus. Les éléments spécifiques des tissus, qui ont été identifiés peuvent être également modifiés, par exemple par mutagénèse de site. Les éléments de régulation modifiés peuvent être ensuite testés pour leur aptitude à commander l'expression génique spécifiques dans des tissus et identifiant ainsi les autres séquences, qui confèrent la spécificité dans les tissus. Ces techniques pour l'identification des éléments de régulation sont applicables à tous les gènes de tubuline α de maïs. Par exemple, de manière préférée, une analyse de l'ERA du gène de tubuline α de maïs *Tub* α *1* indique que les éléments de régulation, qui commandent l'expression génique spécifique des racines, sont constitués par les nucleotides 963 à 1115 et 1 à 1115 de la séquence representée ID N°:1.

D'autres éléments de régulation selon l'invention commandent l'expression génique spécifique du pollen. L' expression génique spécifique du pollen est d'un intérêt et d'une importance particuliers. Normalement le gène de tubuline α de maïs *Tub* α *1* est exprimé à la fois dans les racines et le pollen. Lorsque des régions particulières du gène de tubuline α de maïs *Tub* α *1* sont isolées de l'ERA entier selon l'invention, l'expression est exclusivement localisée dans le pollen. Un élément de régulation spécifique du pollen comprend une séquence de nucléotide particulière, qui est capable de déclencher l'expression d'un gène sous son contrôle dans le pollen, c'est à dire pour le produit du gène détecté dans le pollen à l'exclusion d'autres tissus. Les éléments de régulation qui commandent l'expression spécifique du pollen sont identifiés par analyse des fragments de gène de tubuline α de maïs pour leur aptitude à commander l'expression génique spécifiques du pollen , comme décrit ci-dessus pour l'identification des éléments de régulation spécifiques des racines sauf que l'expression est decelée dans le pollen. Les modifications des séquences de nucléotides permettant l'expression spécifique du pollen sont identifiés comme décrit ci-dessus. Les éléments de régulation spécifiques du pollen issus de tout gène de tubuline α de maïs peuvent être identifiés par ces techniques. Par exemple, de manière préférée, une analyse de l'ERA du gène de tubuline α de maïs *Tub* α *1* indique que les éléments de régulation, qui commandent l'expression génique spécifique du pollen, sont constitués par les nucleotides 1 à 1348 et 1295 à 1348 de la séquence représentée SEQ ID N°:1.

D'autres éléments de régulation selon l'invention, se trouvant dans les régions de l'ERA du gène de tubuline α de maïs, commandent l'expression génique spécifique des méristèmes. Les éléments de régulation qui confèrent l'expression génique spécifique des méristèmes sont identifiés comme décrit ci-dessus pour l'identification des autres éléments de régulation. Par exemple, on peut utiliser l'analyse par délétion pour identifier les séséquences de nucléotides de tout gène de tubuline α de maïs qui commande l'expression d'un gène sous son contrôle dans les méristèmes. De telles séquences peuvent être modifiées comme décrit ci-dessus et testées pour identifier d'autres séquences qui confèrent l'expression génique spécifique des méristèmes. Par exemple, de manière préférée, une analyse de l'ERA du gène de tubuline α de maïs *Tub* α *3* indique que les éléments de régulation, qui commandent l'expression génique spécifique du pollen, sont constitués par les nucléotides 1 à 695 et 542 à 695 de la séquence représentée SEQ ID N°:3.

D'autres éléments de régulation selon l'invention, se trouvant dans les régions de l'ERA du gène de tubuline α de maïs, commandent l'expression génique spécifique des embryons immatures. Les éléments de régulation qui confèrent l'expression génique spécifique des embryons immatures. sont identifiés comme décrit ci-dessus pour l'identification des autres éléments de régulation. Par exemple, on peut utiliser l'analyse par délétion pour identifier les séquences de nucléotides de tout gène de tubuline α de maïs qui commande l'expression d'un gène sous son contrôle dans les embryons immatures. De telles séquences peuvent être modifiées comme décrit ci-dessus et testées pour identifier d'autres séquences qui confèrent l'expression génique spécifique des embryons immatures. Par exemple, de manière préférée, une analyse de l'ERA du gène de tubuline α de maïs *Tub α 3* indique que les éléments de régulation, qui commandent l'expression génique spécifique des embryons immatures, sont constitués par les nucleotides 1 à 1076 de la séquence représentée SEQ ID N°:3.

On peut obtenir un acide nucléique isolé codant pour l'ensemble de régulation amont d'un gène de tubuline α de maïs de la façon suivante. On isole des clones génomiques recombinants de tubuline α par criblage dans une bibliothèque d'ADN génomique de maïs avec un ADNc de la tubuline α ( Montoliu *et al.*, 1989). Des méthodes utiles pour obtenir l'ADN recombinant de la tubuline α sont décrites dans Ausubel *et al.* 1989, Currents protocols in Molecular Biology, John Wiley & Sons, NY, par exemple ou un quelconque des très nombreux manuels sur la technologie de l'ADN recombinant, qui sont largement accessibles. Pour la détermination de séquences de nucléotides une multitude de techniques sont disponibles et connues de l'homme de métier. Par exemple, l'ERA du gène de tubuline α peut être sous cloné en un site polylinker d'un vecteur de séquençage tel que pBluescript (Stratagene). Ces sous clones pBluescript peuvent être ensuite séquencés par la méthode "Dideoxy double brin" (Chen and Seeburg, 1985 DNA 4, 165).

La séquence de nucléotides pour l'ADN codant pour le clone *Tub α 1* de l'ERA du gène de tubuline α de maïs est représentée comme SEQ ID N°: 1.

La séquence de nucléotides pour l'ADN codant pour le clone *Tub α 2* de l'ERA du gène de tubuline α de maïs est représentée comme SEQ ID N°: 2.

La séquence de nucléotides pour l'ADN codant pour le clone *Tub α 3* de l'ERA du gène de tubuline α de maïs est représentée comme SEQ ID N°: 3.

La séquence de nucléotides pour le de peptide de transit optimisé est représentée comme SEQ ID N°: 4.

Les ERAs d'autres gènes de tubuline α de maïs peuvent être obtenus selon la même stratégie. Par ailleurs des clones représentatifs d'autres membres de la famille de gènes de tubuline α de maïs peuvent être obtenus en utilisant les séquences transcrites de l'ERA des *Tub α 1*, *Tub α 2* et *Tub α 3* comme sondes d'hybridation pour cribler une bibliothèque d'ADN génomique de maïs et identifier d'autres gènes de tubuline α.

L'identification de séquences régulatrices cis qui commandent l'expression génique spécifique dans des tissus peut être effectuée par des fusions transcriptionnelles de séquences spécifiques avec la séquence codante d'un gène hétérologue, transfert du gène chimère dans un hôte approprié et détection de l'expression du gène hétérologue. Le test utilisé pour déceler l'expression dépend de la nature de la séquence hétérologue. Par exemple des gènes rapporteurs, tels que celui de la β-glucuronidase (GUS) est communément utilisé pour constater la compétence transcriptionelle et traductionnelle de la construction chimère. Des tests normalisés sont disponibles pour déceler de manière sensible l'enzyme rapporteur dans un organisme transgénique. Le gène GUS est utile comme rapporteur d'une activité promotrice dans les plantes transgéniques en raison de la grande stabilité de l'enzyme dans les cellules végétales et la disponibilité d'un test fluorométrique quantitatif et d'une technique de localisation histochimique. Jefferson *et al.*, 1987 EMBO J 6, 3901 ont mis au point des procédures normalisées pour la détection biochimique et histochimique de l'activité GUS dans les tissus de plantes. Les tests biochimiques sont effectués en mélangeant des lysats de tissus de plantes avec le 4-méthylombelliferyl-β-D-glucuronide, un substrat fluorométrique pour pour GUS, puis incubation une heure à 37°C puis mesure de la fluorescence de la 4-méthylombelliférone. La localisation histochimique de l'activité GUS est déterminée par incubation des échantillons de tissus de plantes dans le 5-bromo-4-chloro-3-indolyl-glucuronide(X-Gluc) pendant 18 heures à 37°C et observation du patron de taches de X-Gluc. La construction de tels gènes chimères permet une définition des séquences de régulation spécifiques nécessaires pour la régulation de l'expression et démontre par l'analyse que ces séquences peuvent commander l'expression de gènes hétérologues.

La présente invention comprend également un gène chimère végétal contenant un élément de régulation issu d'un gène de tubuline α de maïs, qui commande l'expression génique spécifique dans les racines, l'expression génique spécifique dans le pollen, l'expression génique spécifique dans le méristème ou l'expression génique spécifique dans les embryons immatures et relié à la séquence codante d'un gène hétérologue de sorte que l' élément de régulation soit capable de contrôler le gène hétérologue. Le gène hétérologue peut être tout gène autre que le gène de tubuline α de maïs. Si nécessaire, on peut inclure dans les constructions chimères d'autres éléments promoteurs, en totalité ou en partie, tels que des introns de monocots suffisant pour que leur expression produise une quantité efficace du polypeptide codé par le gène hétérologue et apportant toute propriété agronomique utile telle que la résistance aux insectes, aux nématodes, aux champignons et de préférence aux herbicides.

Par conséquent la présente invention comprend des gènes chimères comprenant des régions de l'ERA de la tubuline α de maïs, qui confère une expression spécifique des racines selon l'invention, qui sont reliés à une séquence codant pour une enzyme de résistance herbicide. De préference les régions de l'ERA comprennent les nucléotides 1 à 1115, 963 à 1115 ou 1 à 1529 de *Tub* α *1*, comme indiqué dans SEQ ID N°: 1. Toute modification de ceux-ci conférant une expression spécifique des racines fait partie de l'invention. Les racines accumulent certaines classes d'herbicides, qui les rendent très sensibles à des applications de ces herbicides à faible doses. Comme les éléments de l'ERA de la tubuline α de maïs peuvent commander une expression élevée, régulée dans les racines, ils sont utiles pour donner un phénotype de résistance. Ces éléments sont utiles pour réguler l'expression de gènes codant pour des enzymes de résistance herbicide tels que le gène *aroA de S. Thyfimurium* et les enzymes de détoxyfication des herbicides, tels que le gène *brx de K. ozaenae* résistant au bromoxynil. On peut utiliser des gènes chimères contenant ces éléments pour produire des lignées de plantes transgéniques résistantes à des doses agronomiques d'herbicides.

L'invention comprend également des gènes chimères ayant une région de l'ERA de la tubuline α de maïs, qui confère une expression spécifique du pollen et fusionné avec un gène hétérologue. Cette construction confère une expression spatialement disitncte de l'expression "normale" de la tubuline α de maïs en ce que le gène hétérologue est exprimé directement dans le pollen végétal. Autrement dit lorsqu'une séquence spécifique est enlevée du context de l'ERA entier la régulation spécifique du tissu est modifiée. De préference les régions de l'ERA de *Tub* α *1* comprennent les nucléotides 1 à 1348 ou 295 à 1348, comme indiqué dans SEQ ID N°: 1. De préférence également cet élément de régulation peut être fusionné avec des protéines cytotoxiques pour créer des plantes stériles males. Toute modification de ceux-ci conférant une expression spécifique du pollen fait partie de l'invention.

L'invention comprend également des gènes chimères ayant une région de l'ERA de la tubuline α de maïs, qui confère une expression spécifique des méristèmes et fusionné avec un gène hétérologue. De préférence les régions de l'ERA de *Tub* α *3* comprennent les nucléotides 1 à 695 ou 542 à 695, comme indiqué dans SEQ ID N°: 3. Toute modification de ceux-ci conférant une expression spécifique des méristèmes fait partie de l'invention.

L'invention comprend également des gènes chimères ayant une région de l'ERA de la tubuline α de maïs, qui confère une expression spécifique des embryons immatures et fusionné avec un gène hétérologue. De préférence les régions de l'ERA de *Tub* α *3* comprennent les nucléotides 1 à 1076, comme indiqué dans SEQ ID N°: 3. Toute modification de ceux-ci conférant une expression spécifique des embryons immatures fait partie de l'invention.

L'utilisation de ces constructions chimères est particulièrement importante pour apporter de la résistance herbicide. Comme la plupart des herbicides de font pas de différence entre les adventices et les cultures, le génie génétique des plantes de culture résistantes aux herbicides est d'une importance agronomique considérable dans la mesure où il permet d'utiliser des herbicides à large spectre. Par conséquent, la présente invention comprend des gènes chimères comprenant des éléments de l'ERA de la tubuline α de maïs, qui confèrent une expression spécifique des racines à au moins une partie d'un promoteur, qui fonctionne dans les plantes et qui de plus est fusionné avec au moins une partie du gène *aroA* ou une séquence codant pour un polypeptide conférant de la résistance herbicide. Comme exemples on peut citer des polypeptides conférant de la résistance au glyphosate, et aux inhibiteurs voisins de la 5-énolpyruvylshikimic acid-3-phosphate synthase (EPSPS), sulfonylurées, imidazolinones et les inhibiteurs de l'acétoxhydroxy acid synthase(AHS), et de la 4-hydroxyphényl pyruvate dioxygénase(HPPO). De préférence les régions de l'ERA de *Tub* α *1* comprennent les nucléotides 1 à 1115, 963 à 1115 ou 1 à 1529 comme indiqué dans SEQ ID N°: 1 et sont fusionnés au gène rapporteur. Toute modification de ceux-ci conférant une expression spécifique des embryons immatures fait partie de l'invention.

Les gènes chimères selon l'invention sont construits par fusion d'une séquence promotrice, partielle ou entière, de tubuline α de maïs avec la séquence codante d'un gène hétérologue. La juxtaposition de ces séquences peut être effectuée de multiples manières. De préférence, l'ordre des séquences, de 5' à 3' s'établit ainsi: ERA de la tubuline α de maïs, une séquence d'intron de monocot ou de dicot, par exemple de tabac, une séquence codante et un site de polyadénylation.

Les méthodes classiques de constructions de tels gènes chimères sont bien connues de l'homme de métier et peuvent être trouvées dans des références telles que Ausubel *et al.*(1989). Diverses stratégies sont disponibles pour ligaturer les fragments d'ADN, le choix dépendant de la nature des terminaisons des fragments d'ADN. L'homme de métier sait que pour que le gène hétérologue puisse s'exprimer, la construction nécessite des éléments promoteurs et de signaux pour une polyadénylation efficace du transcrit. Par conséquent, les régions de l'ERA de la tubuline α de maïs qui contiennent les séquences promotrices connus comme les boites CAAT et TATA peuvent être fusionnées directement avec une séquence codante sans promoteur. Par ailleurs, les régions de l'ERA de la tubuline α de maïs qui ne contiennent pas de boites CAAT et TATA peuvent être reliées à un fragment d'ADN codant pour un promoteur, qui fonctionne dans les plantes. Des promoteurs de plantes peuvent être trouvés dans le commerce ou être synthéthisés chimiquement en se référant à leur séquence publiée. Comme exemple d'un tel fragment on peut citer le promoteur 35S tronqué du virus de la mosaïque du choux-fleur, qui garde ses boites CAAT et TATA. On peut utiliser d'autres promoteurs comme ceux de la nopaline synthase et la ribulose 1,5 bisphosphate carboxylase. Le fragment promoteur est ensuite relié à la séquence codante hétérologue. L'extrémité 3' de la séquence codante est fusionnée avec un site de polyadénylation par exemple et de manière non limitative, celui de la nopaline synthase. En outre on peut utiliser des vecteurs de transformation des plantes qui contiennent un ou plusieurs sites de polyadénylation entourés de séquences requises pour la transformation des plantes.

Les éléments de l'ERA de la tubuline $\alpha$ de maïs et les séquences codantes hétérologues de l'invention peuvent être sous clonées dans un site polylinker d'un vecteur de transformation des plantes pour obtenir les gènes chimères.

Les éléments 5' de la présente invention peuvent être dérivés de la digestion d'un clone génomique de tubuline $\alpha$ de maïs par une endonuclease de restriction ou une exonuclease. Les fragments de restriction contenant les boites CAAT et TATA sont ligaturés avec un gène hétérologue sans promoteur tel qu'une séquence codante de GUS ou d'*aroA*. L'homme de métier sait que les éléments de régulation 5' de tubuline $\alpha$ de maïs peuvent être obtenus par d'autres moyens, par exemple synthèse chimique ou enzymatique(PCR). Le produit hétérologue peut être la séquence codante de tout gène, qui peut être exprimé dans une telle construction. Tous ces exemples font partie de l'invention. L'extrémité 3' de la séquence codante est éventuellement fusionnée avec un site de polyadénylation, tel que et de manière non limitative, celui de la nopaline synthase, celui du gène 7 de l'ADN-T de l'octopine ou celui du gène H4A748 du gène d'histone *d'Arabidopsis*. Par ailleurs, le site de polyadénylation peut être fourni par le gène hétérologue lui même.

Les éléments de régulation 5' de la tubuline $\alpha$ de maïs qui ne contiennent pas de boîte TATA peuvent être reliés à au moins une partie de la séquence de promoteur de plante, c'est à dire contenant au moins les séquences CAAT et TATA. De préférence ce promoteur est le promoteur 35S tronqué du virus de la mosaïque du choux-fleur. Le complexe chimère résultant peut être ligaturé avec une séquence codante hétérologue et une séquence de polyadénylation.

Pour obtenir une expression régulée des gènes hétérologues, les plantes sont transformées avec les gènes chimères selon l'invention. Le transfert de gène est bien connu de la technique comme une méthode d'expression de gènes hétérologues dans les plantes transgéniques. Le tabac est la plante le plus souvent utilisée en raison de sa facilité de régénération, le grand rendement en semences par plante et qu'il peut être transformé avec une grande fréquence avec les vecteurs dérivés de plasmides Ti d'*Agrobacterium* (Klee et al. (1987) Annu. Rev. Plant Physiol. 38, 467). Les plantes dicotyledones telles que, de manière non limitative, le coton, le colza, et le soja sont les hôtes transgéniques préférés. Cependant il sera à la portée de l'homme de métier que toute plante peut être efficacement transformée et régénérée comme hôte transgénique selon l'invention.

De nombreuses méthodes de transformation sont connues. Les gènes chimères peuvent être introduits par un processus de transformation sur disque foliaire et régénération comme décrit par (Horsch *et al.* (1985) Science 227, 1229). D'autres méthodes de transformation, telles que la culture de protoplasts( Horsch *et al.* (1984) Science 223, 496; DeBlock *et al.* (1984) EMBO J 2, 2143; Barton *et al.* (1983) Cell 32, 1033) ou la transformation in vitrod'explantes de tiges ou de racines( Zambrisky *et al.* (1983) EMBO J 2, 2143; Barton *et al.* (1983) Cell 32, 1033) peuvent aussi être utilisées dans le cadre de l'invention. De préférence les plantes sont transformées avec des vecteurs dérivés d' *Agrobacterium*. Cependant d'autres méthodes sont disponibles pour insérer les gènes chimères de l'invention dans les cellules de plantes. Parmi ces méthodes on peut citer les approches par la biolistique(Klein *et al.* (1983) Nature 327 70), l'électroporation, l'absorption d'ADN par induction chimique et l'utilisation des virus ou des pollens comme vecteurs.

Si nécessaire pour la méthode de transformation, les gènes chimères selon l'invention peuvent être introduits dans un vecteur de transformation des plantes, par exemple le vecteur binaire décrit par Bevan (1984) ou une autre méthode décrite dans la demande européenne 337 899. Les vecteurs de transformation des plantes peuvent être dérivés par modification du système de transfert naturel de gène d'*Agrobacterium tumefaciens*. Le système naturel comprend de grands plasmides Ti (Tumor inducing) contenant un grand segment, appelé ADN-T, qui est transferré aux plantes transformées. Un autre segment du plasmide Ti, la région vir est responsable du transfert de l' ADN-T. L' ADN-T est entouré par des répétitions terminales. Dans les vecteurs binaires modifiés, les gènes qui induisent la tumeur ont été délétés et les fonctions de la région vir sont utilisées pour pour transférer l'ADN étranger bordé par les séquences limites ADN-T. La région T contient aussi un marqueur de sélection pour la résistance à un antibiotique, et un site de clonage multiple pour l'insertion des séquences à transférer. ces souches de génie génétique sont connues sous le nom d'*Agrobacterium tumefaciens* "désarmées"et permettent une transformation efficace des séquences bordées par la région T dans le génome nucléaire des plantes.

Les disques foliaires stérilisés en surface sont inoculés avec d'*Agrobacterium tumefaciens* contenant l'ADN étranger, cultivés pendant deux jours puis transférés dans un milieu contenant un antibiotique. Les pousses transformées sont choisies après un racinage dans un milieu contenant l'antibiotique approprié et transférés en terre. Les plantes transgéniques sont autopollinisées et les semences de ces plantes récoltées et cultivées dans un milieu contenant l'antibiotique.

L'expression d'un gène rapporteur ou hétérologue dans les racines, les pousses, le pollen, les méristemes et les embryons immatures peut être contrôlée par des tests immunologiques, histochimiques ou d'activité.

Comme on le verra ci-après, le choix d'un test d'expression du gène chimère dépend de la nature de la région codante hétérologue. Par exemple, l'analyse Northern peut être utilisée pour suivre la transcription si les sondes nucléotidiques appropriées sont disponibles. Si on dispose d'anticorps pour le polypeptide codé par le gène hétérologue, on peut utiliser l'analyse Western et la localisation immuno-histochimique pour suivre la production et la localisation du polypeptide. Selon le gène hétérologue, on peut utiliser les tests biochimiques. Par exemple, les acétyltransférases sont décelées par mesure de l'acétylation d'un substrat standard. L'expression d'un gène de résistance herbicide peut être suivie par détermination de la résistance herbicide de la plante transformée.

L'invention comprend également des plantes transgéniques tant monocotylédones que dicotylédones et leur descendance contenant les gènes chimères selon l'invention. Les cellules végétales sont transformées avec les gènes chimères par toute méthode de transformation des plantes décrites ci-dessus. La cellule végétale transformée, habituellement dans une culture de cal ou de disque foliaire, est régénérée en une plante transgénique complète par des méthodes bien connues de l'homme de métier (p.e. Horsch *et al.* (1985) Science 227, 1129). De préference, la plante transgénique est le coton, le colza, le maïs, le tabac ou le soja. Comme la descendance des plantes transformées transmet le gène chimère, les semences ou les explantes issus des plantes transformées sont utilisées pour maintenir la lignée de plantes transgéniques.

L'invention comprend également une méthode de production d'une plante avec une résistance herbicide améliorée. Cette méthode comprend la transformation d'une cellule végétale avec un vecteur contenant un gène chimère comprenant un élément de régulation, spécifique des racines ou des méristèmes, relié à la séquence codante d'une enzyme de résistance herbicide ou d'une enzyme dégradant un herbicide et sélection d'une plante avec les propriétés désirées. De préference les éléments sont des régions de l'ERA de *Tub* α *1* comprennent les nucléotides 1 à 1115, 963 à 1115 ou 1 à 1529 comme indiqué dans SEQ ID N°: 1. Les plantes transformées sont régénérées en plantes qui sont résistantes à une application herbicide.

L'invention comprend également une méthode de production d'une plante male stérile. Cette méthode comprend la transformation d'une cellule végétale avec un vecteur contenant un gène chimère comprenant un élément de régulation, spécifique du pollen, relié à la séquence codante d'une protéine cytotoxique et sélection d'une plante avec les propriétés désirées. De préference les éléments sont des régions de l'ERA de *Tub* α *1* comprenant les nucléotides 1 à 1348 ou 1295 à 1348 comme indiqué dans SEQ ID N°: 1. Les plantes transformées sont régénérées en plantes qui sont males stériles.

L'invention comprend également une méthode de production d'une plante résistante à un herbicide. Cette méthode comprend la transformation d'une cellule végétale avec un vecteur contenant un gène chimère comprenant un élément de régulation, spécifique des racines, relié à la séquence codante d'un gène de résistance à un herbicide tel que la N-phosphonométhylglycine les plantes avec la résistance herbicide désirée étant ensuite sélectionnées. Les plantes sélectionnées sont celles qui survivent à un traitement herbicide qui tue les plantes non transformées de la même espèce dans les mêmes conditions. De préference les éléments sont des régions de l'ERA de *Tub* α *1* comprennent les nucléotides 1 à 1115, 963 à 1115 ou 1 à 1529 comme indiqué dans SEQ ID N°: 1 et la séquence hétérologue est fournie par un gène codant pour la EPSPS synthase, l'acétolactase synthase ou la 4-hydroxyphénylpyruvate dioxygénase ou présentant des mutations les rendant résistantes. Les plantes transformées sont régénérées en plantes qui sont résistantes à un herbicide. De préférence les plantes sont transformées par un vecteur pRPA-RD-65 ou pRPA-RD-88) qui contient l'élément de régulation spécifique des racines comprenant les nucléotides 70 à 1529 de l'ERA de *Tub* α *1*, un intron de monocot (pRPA-RD-88 seulement), un peptide de transit optimisé et un gène de résistance herbicide *aroA*.

Les exemples suivants illustrent plus complètement l'invention.

Les séquences de nucléotides auxquelles on fait référence dans les exemples sont numérotées SEQ ID N° 1 à 4.

La Fig. 1 représente la construction du plasmide parental de p. BI 101.1

La Fig. 2 est une représentation schématique de constructions chimères *Tub* α *1*/ promoteur- GUS. Les nombres sont donnés par rapport au site de départ de la transcription. La figure supérieure correspond à la région intergénique entre *Tub* α *1* et *Tub* α *2* et les délétions ont été faites par restriction aux sites appropriés.La figure inférieure correspond au fragment -449 où les délétions ont été faites par digestion à plusieurs reprises avec l'Exonuclease III. Le site SacII(+ 48) corresponds au point de fusion transcriptioonnel entre le promoteur et le plasmide pBI 101.1 . (+) correspond au site de départ de la transcription du gène de tubuline *Tub* α *1*.

La Fig. 3 est une représentation schématique de constructions chimères *Tub* α *3*/ promoteur- GUS. Les nombres sont donnés par rapport au site de départ de la transcription. Les délétions ont été faites par digestion à plusieurs reprises avec l'Exonuclease III. Le site BglII corresponds au point de fusion transcriptioonnel entre le propmoter et la site BamH1 du plasmide pBI 101.1. (+) correspond au site de départ de la transcription du gène de tubuline *Tub* α *3*.

Exemple 1:

a) Constructions avec le gène rapporteur GUS:

Les cassettes à usage général du gène rapporteur GUS utilisées dans les exemples ont été décrites par Jefferson *et al.*, 1987 EMBO J 6, 3901. En résumé, la séquence codante de GUS a été ligaturée avec la partie 5' du site de polyadénylation de la nopaline synthase dans le site polylinker du vecteur pBIN19 dérivé de *A.tumefaciens* (Bevan(1984) Nucleics Acids Res. 12, 8711). Le vecteur pBIN19 contient les bordures gauche et droite d'ADN-T nécessaire pour la transformation végétale, et un gène de résistance à la kanamycine. La construction résultante, pBI101.1 est décrite à la Fig.1. Seuls les sites de restriction amont du codon d'initiation AUG de GUS permettent l'insertion des fragments d'ADN promoteurs .

Le Tableau 1 décrit les plasmides parentaux et les constructions dérivées. Le *Tub* α *1* de maïs et les positions des éléments promoteurs contenus dans 1s constructions dérivées sont représentées à la Fig. 2.

Les constructions p*Tub* α *1- GUS* représentent de grands fragments de recouvrement qui enjambent toute la longueur de la région régulatrice ( -1410 à 48 de la Fig. 2). Les extrémités 5' de plusieurs constructions ont été dérivées de digestions à l'exonucléase III d'un fragment de 497 pb de *Tub* α *1* dans pBI101.1 (*Stratagene*) [-449(SpcI-SacII), Tableau 1]. La position de chaque délétion est montrée à la Fig. 2.

b) Transformation des plantes

Les constructions de plasmides à base de pBIN19 ont été utilisées pour transformer du tabac (Nicotinia tabacum cv petite Havana SR1) selon les procédures classiques(Horsch *et al.* (1985) sauf que les transformants initiaux ont été sélectionnés sur 100μg/ml de kanamycine.

Les plantes ont été autopollinisées, et les gaines de F1 mises en germination sur 200g/ml de kanamycine pour identifier les transformants, puisque les constructions à base de pBIN19 contiennent le gène de la néomycine phophotransférase(NPTII), qui confère la résistance à l'antibiotique toxique kanamycine. Le nombre de copies de chaque construction GUS intégré dans le génome du tabac a été estimé pour chaque transformant par analyse des fréquences de ségrégation de la résistance à la kanamycine. La plupart des transformantscontenaient seulement un locus ségrégant de la construction. On a fait pousser les plantes transgéniques en serre.

TABLEAU 1

| Construction Plasmides parentaux | Description |
|---|---|
| pBI101.1 | pBIN-19 cassette du gène, sans promoteur, dérivé du gène rapporteur GUS(Jefferson *et. al.*, 1987)(cf Fig. 1). |
| pRPA-BL-504 | semblable à pBI101.1 sauf que le gène pour la chloramphenicol acetyltransferase remplace le gène GUS . |
| p*Tub*α *1* | fragment XhoI de 3058 pb de MG19/6 (Montoliu *et. al.*, 1989) cloné dans pUC-18; contient la totalité de l'ensemble de régulation amont de *Tub*α *1* de maïs. |
| p*Tub*α *1*-1588 | fragment XhoI-AluI de1588 pb de *Tub*α *1* de maïs (Montoliu *et. al.*, 1989) cloné dans pUC-18; contient 1410 pb en amont et 180 pb en aval du site de départ de la transcription . |
| p*Tub*α *3*-1072 | fragment Bgl II de1072 pb de *Tub*α *3* de maïs (Montoliu *et. al.*, 1990) cloné dans le site BamHI de pUC-18; contient 1020 pb en amont et 62 pb en aval du site de départ de la transcription . |
| pRPA-RD-37B | cassette d'expression de *aroA* contenant un peptide de transit optimisé fusionné dans le cadre (EP 508 909), un gène *aroA* et un signal de polyadenylation NOS cloné dans pBS II SK(-) (Stratagene). |
| pRPA-RD-49 | Vecteur de transformation de monocot contenant un promoteur CaMV 35S , un gène *bar* et un signal de polyadenylation TR7 cloné entre les sites HindIII et EcoRI de pUC-18. Un linker NotI a été ligaturé à extrémité franche entre les deux sites AflIII and SspI sites créant un fragment NotI de 3.1 kb contenant la construction de gène CaMV 35S - *bar* . |
| pRPA-RD-26 | fragment EcoRI - ScaI de1865 pb de p*Tub*α *1* cloné dans pUC-19 digéré EcoRI - HincII. La séquence entourant l'ATG d'initiation de traduction a été mutée par PCR pour contenir un site NcoI site en |

utilisant le primer 5'->3' C GGC CGC CGC TCC ACC CGT ACG ACG ACC ACC ATG GGG GAG.

| | |
|---|---|
| pRPA-RD-32 | un fragment PstI - NcoI de 1458 pb de pRPA-RD-26 constitué des nucleotides -1340 à 118 issu du gène de maïs *Tubα 1* (nucleotides 71 à 1527 de SEQ ID NO. 1) a été ligaturé dans les sites PstI - NcoI sites de pRPA-RD-37B créant une cassette d'expression *Tubα 1* de maïs-OTP - gène *aroA* - NOS . |
| pRPA-RD-87 | un fragment NcoI - PstI de 600 pb contenant l'intron 1 *adhI* de maïs a été cloné dans les sites NcoI - PstI de pRPA-RD-37B créant une cassette d'expression: intron 1 *adhI* de maïs - peptide de transit optimisé (EP 508 909), gène *aroA* et signal de polyadenylation NOS. |
| pRPA-RD-90 | un fragment EagI de 1430 pb issu de pRPA-RD-32 (contenant nucléotides -1340 to 90 du gène de maïs *Tubα 1* ; sequence 71 à 1499 of SEQ ID NO. 1) coupé avec extrémité franche par le fragment de Klenow de l'ADN polymérase I d'ADN de E. coli cloné dans le site SmaI de pBS II SK(-) (Stratagene). |

Constructions dérivées

| | |
|---|---|
| -1410 | fragment HindIII - SacII de 1474 pb de p*Tubα 1*-1588 cloné dans pBI101.1 digéré HindIII - SmaI; contient 1410 pb en amont et 48 pb en aval du site de départ de la transcription . |
| *Tubα 3*-1020 | fragment HindIII - SmaI de1115 pb de p*Tubα 3*-1072 cloneé dans pBI101.1 digéré HindIII - SmaI; contient 1020 pb en amont et 62 pb en aval du site de départ de la transcription(cf Fig. 2) . |
| -956 | un dérivé de -1410 généré par digestion BamHI et recircularisation; contient 956 pb en amont et 48 pb en aval du site de départ de la transcription . |
| -449 | un dérivé de -1410 généré par digestion SpeI et recircularisation; contient 449 pb en amont et 48 pb en aval du site de départ de la transcription . |

| | |
|---|---|
| -352 | un dérivé de -449 (dans pBI101.1) généré par digestion exonuclease III et recircularisation; contient 352 pb en amont et 48 pb en aval du site de départ de la transcription . |
| -297 | un dérivé de -449 (dans pBI101.1) généré par digestion exonuclease III et recircularisation; contient 297 pb en amont et 48 pb en aval du site de départ de la transcription . |
| -252 | un dérivé de -449 (dans pBI101.1) généré par digestion exonuclease III et recircularisation; contient 252 pb en amont et 48 pb en aval du site de départ de la transcription . |
| -184 | un dérivé de -449 (dans pBI101.1) généré par digestion exonuclease III et recircularisation; contient 184 pb en amont et 48 pb en aval du site de départ de la transcription . |
| -117 | un dérivé de -449 (dans pBI101.1) généré par digestion exonuclease III et recircularisation; contient 117 pb en amont et 48 pb en aval du site de départ de la transcription . |
| -64 | un dérivé de -449 (dans pBI101.1) généré par digestion exonuclease III et recircularisation; contient 64 pb en amont et 48 pb en aval du site de départ de la transcription . |
| pRPA-RD-53 | un fragment SacI - EcoRI de 3.5 kb contenant la cassette d'expression *Tubα 1* de maïs- *aroA* de pRPA-RD-32 cloné dans les sites SacI - EcoRI de pBIN-19. |
| pRPA-RD-65 | un fragment SacI - EcoRI de 3.5 kb contenant la cassette d'expression *Tubα 1* de maïs- *aroA* de pRPA-RD-32 rendu à extrémité franche par la polymerase T4 DNA cloné dans le site NdeI de pRPA-RD-49 qui a été coupé avec extrémité franche par le fragment Klenow de l'ADN polymerase d'E. coli. l'orientation de la cassette d'expression *Tubα 1* de maïs- *aroA* de pRPA-RD-32 s'est avérée divergente par rapport aux unités de transcription du gène *aroA* et *bar*. |

pRPA-RD-88 un fragment PstI de1.5 kb de pRPA-RD-90 contenant nucléotides -1340 à 90 du gène *Tub*α *1* de maïs (séquence 71 à 1499 de SEQ ID N°. 1) cloné dans le site PstI de pRPA-RD-87 avec création d'une cassette d'expression: *Tub*α *1* de maïs - intron 1 *adhI* de maïs - peptide de transit optimisé (EP 508 909), gène *aroA* et signal de polyadénylation NOS.

pRPA-RD-7: un dérivé du peptide de transit optimisé (EP 508 909; SEQ ID NO: 4) qui a été muté par PCR avec les oligonucleotides synthétiques suivants: (1) GAA TTC CGA AAG ACA AAG ATT ATC GCC ATG GCT TCG [nucleotides 1-36; SEQ ID NO: 3]; (2) CCG TAG GCC GGC CAC ACC TGC ATA CAT TGA ACT CTT CC [nucleotides 228-181; SEQ ID NO: 3]; and (3) CGA GAC GCT GTC GTA CCT GCC GCC GCT GTC TAT GGC G [nucleotides 231-267; SEQ ID NO: 3]. Ce peptide de transit optimisé a été cloné dans les sites EcoRI et EcoRV de pBSII SK(-) [Stratagene] avec création d'une cassette de clonage contenant le peptide de transit optimisé.

Exemple 2: localisation histochimique de l'activité GUS: expressions spécifiques des racines et du pollen

L'activité GUS a été déterminée dans les racines, le pollen et divers autres tissus de tabac transgénique contenant chacune des constructions et réprésentée au tableau 1. On a suivi les procédures classiques de Jefferson *et al.* (1987).

L'activité GUS a été localisée de manière histochimique dans des plantes transgéniques contenant des gènes GUS chimères utilisant des fragments promoteurs issus du gène *Tub*α *1* de maïs. Des échantillons ont été lavés dans un mélange de 50mM $NaPO_4$, pH 7, 0,2mM 5-bromo-4-chloro-3-indolyl-glucuronide(X-Gluc), .,1 mM ferricyanure de potassium et 0,1mM de ferrocyanure de potassium. Les échantillons ont été montés sur des lamelles de microscope recouvertes de 80% de glycérol.

Le tableau 2 présente en résumé les résultats de microphotos typiques de plantes de tabac contenant des éléments de régulation du gène *Tub*α *1* de maïs. Le tableau 2 montre que les éléments de régulation de *Tub*α *1* donnent des niveaux élevés d'expression dans les tissus méristématiques, en particulier les méristèmes de racines et le pollen.

L'activité GUS a été analysée par fluorométrie par broyage du tissu végétal dans un tampon d'extraction(50mM $NaPO_4$, 10 mM EDTA, 0,1% Sarkosyl, 0,1% Triton X-100 et 10 mM de β- mercaptoéthanol). Après centrifugation du lysat, le surnageant est enlevé et mis dans un tube neuf et ajouté par aliquots de 100µl. Un égal volume de 2 mM de 4- met ombelliféryl-β-glucuronide dans le tampon d'extraction est ajouté et mis en incubation à 37°C pendant 1 heure. Les réactions sont arrêteées avec 0,8ml $Na_2$ $CO_3$ (0,2M).La fluorescence de la 4- met ombelliférone (4-MU) a été déterminée avec un minifluoromètre de Hoeffer comme décrit par Jefferson *et al.* (1987). L'activité GUS est exprimée en picomoles 4-MU par unité de la masse totale de protéine par minute.Pour déterminer les éléments promoteurs responsables de la spécificité méristèmes vis à vis de l'expression spécifique du pollen, le patron d'expression de GUS de diverses délétions de promoteurs(cf Fig. 2) a été déterminé dans les plantes de tabac transgéniques.

Des pointes de racines, des pointes de pousses, des feuilles, des tiges et du pollen issus de plantes transgéniques contenant divers éléments de séquence de *Tub*α *1*(résumé à la Fig. 2) dirigeant l'expression de GUS ont été testés par rapport à l'activité.Les résultats sont présentés au tableau 3. Toutes les constructions contenant une portion de l'ERA du gène *Tub* α de la tubuline α du maïs entre -1410 et -352(SEQ ID N° 1:1 à 1058) ont conférés une activité de GUS dans les racines de tabacs transgéniques. La longueur totale de la région régulatrice et les fragments qui en dérivent confèrent tous une importante activité dans les protopiastes de tabac. Ce n'est qu'après délétion d'éléments promoteurs plus proches que 352 pb en amont du site du départ de la transcription (1058 de SEQ ID N° 1) que l'activité GUS est supprimée dans les racines transgéniques ce qui montre que les éléments de régulation spécifiques des racines de *Tub*α *1*sont en amont de -352 pb (SEQ ID N° 1:1 à 1058).

Toutes les constructions contenant une portion de l'ERA du gène *Tub*α de la tubuline α du maïs entre -1410 et -64 (SEQ ID N° 1:1 à 1058) ont conférés une activité de GUS dans le pollen de tabacs transgéniques. Ce n'est qu'après délétion d'éléments promoteurs plus proches que 64 pb en amont du site du départ de la transcription (1348 de SEQ ID N° 1) que l'activité GUS est supprimée dans le pollen transgénique, ce qui montre que les éléments de régulation spécifiques du pollen de *Tub*α *1*sont en amont de -64 pb (SEQ ID N° 1: 1 à 1058).

TABLEAU 2: Analyse histochimique de plantes transgéniques .

| | TISSU | | | | |
|---|---|---|---|---|---|
| Construction | pointe de racine | pointe de pousse | feuille | tige | pollen |
| -1410 | + (12/12) | - (12/12) | - (12/12) | - (12/12) | + (12/12) |
| -956 | + (6/6) | - (6/6) | - (6/6) | - (6/6) | + (6/6) |
| -449 | + (8/8) | - (8/8) | - (8/8) | - (8/8) | + (8/8) |
| -352 | - (12/13) | - (13/13) | - (13/13) | - (13/13) | + (6/10) |
| -117 | - (9/9) | - (9/9) | - (9/9) | - (9/9) | + (4/9) |
| -64 | - (11/11) | - (11/11) | - (11/11) | - (11/11) | - (11/11) |

les chiffres indiquent le nombre de plantes avec tache bleue (+) ou sans (-) l'expression par rapport à la totalité des plantes analysées. pour chaque construction dans les diverses parties de la plante.

TABLEAU 3: Test fluorométrique des constructions de GUS avec un promoteur du gène *Tub α 1* la tubuline α de maïs de plantes de tabacs transgéniques.

| | | TISSU | | | | | |
|---|---|---|---|---|---|---|---|
| Construction | jours après germination | racine entière 2 | racine plante témoin 2 | feuilles entières 2 | feuille plante témoin 2 | cotyledon 2 | cotyledon plante témoin 2 |
| -1410 | 10<br>15<br>25 | 388 ± 124<br>727 ± 736<br>265 ± 233 | 15<br>16<br>20 | 39 ± 2<br>61 ± 55<br>15 ± 2 | 13<br>14<br>15 | 148 ± 56 | 15 |
| -956 | 10<br>15<br>25 | 3082 ± 1551<br>4934 ± 557<br>237 ± 170 | 15<br>16<br>20 | 736 ± 499<br>612 ± 363<br>30 ± 15 | 13<br>14<br>15 | 86 ± 52 | 15 |
| -449 | 10<br>15<br>25 | 405 ± 40<br>70 ± 28<br>469 | 15<br>16<br>20 | 73 ± 64<br>118 ± 134<br>19 ± 8 | 13<br>14<br>15 | 172 ± 57 | 15 |
| -352 | 10<br>15<br>25 | 466<br>15<br>26 ± 10 | 15<br>16<br>20 | 127<br>13 ± 2<br>27 ± 2 | 13<br>14<br>15 | 5 ± 2 | 15 |

Exemple 3: Localisation histochimique de l'activité de GUS: expression spécifique des méristèmes

L'activité GUS a été localisée de manière histochimique dans des plantes transgéniques contenant des gènes GUS chimères utilisant des fragments promoteurs issus du gène *Tubα 3* de maïs(nucléotides 1 à 1082 SEQ ID N° 3). Des échantillons ont été lavés dans un mélange de 50mM $NaPO_4$, pH 7; 0,2mM 5-bromo-4-chloro-3-indolyl-glucuronide(X-Gluc);0,1 mM ferricyanure de potassium et 0,1mM de ferrocyanure de potassium. Les échantillons ont été montés sur des lamelles de microscope recouvertes de 80% de glycérol.

Le tableau 4 présente en résumé les résultats de microphotos typiques de plantes de tabac contenant des éléments de régulation du gène *Tubα 3* de maïs. Le tableau 4 montre que les éléments de régulation de *Tubα 3* (nucléotides 1 à 1082 SEQ ID N° 3) donnent des niveaux élevés d'expression de préférence dans les tissus méristématiques.

L'activité GUS a été analysée par fluorométrie par broyage du tissu végétal dans un tampon d'extraction (50mM $NaPO_4$, 10 mM EDTA, 0,1% Sarkosyl, 0,1% Triton X-100 et 10 mM de β- mercaptoéthanol). Après centrifugation du lysat, le surnageant est enlevé et mis dans un tube neuf et ajouté d'aliquots de 100µl. Un égal volume de 2 mM de 4-metombelliféryl-β-glucuronide dans le tampon d'extraction est ajouté et mis en incubation à 37°C pendant 1 heur. Les réactions sont arrêtées avec 0,8ml $Na_2 CO_3$ (0,2M). La fluorescence de la 4-metombelliférone (4-MU) a été déterminée avec un minifluoromètre de Hoeffer comme décrit par Jefferson *et al.* (1987). L'activité GUS est exprimée en picomoles 4-MU par unité de de la masse totale de protéine par minute. Pour déterminer les éléments promoteurs responsables de la spécificité méristèmatiques vis à vis de l'expression spécifique des méristèmes, le patron d'expression de GUS de diverses délétions de promoteurs(cf Fig. 3) a été déterminé dans les plantes de tabac transgéniques.

Tableau 4: Test fluorométrique des constructions de GUS avec un promoteur du gène *Tubα 3* la tubuline α de maïs de plantes de tabacs transgéniques.

| | TISSUE | | | |
|---|---|---|---|---|
| jours après germination | apex de racine | apex de racine plante témoin | apex de pousse | apex de pousse plante témoin |
| 12 | 12,88 ± 6, 68 | 3,65 | 39,02 ± 22,04 | 2,85 |
| 16 | 7,31 ± 3,63 | 3,56 | 33,22 ± 22,00 | 1,93 |
| 19 | 47,67 ± 35,90 | 4,78 | 20,53 ± 4,00 | 3,.06 |
| 23 | 27,17 ± 20,93 | 5,97 | 28,35 ± 10,97 | 0,42 |
| 26 | 31,77 ± 13,81 | 4,82 | 35,66 ± 30,88 | 1,85 |
| 30 | 20,64 ± 12,44 | 8,10 | 29,40 ± 17,88 | 3,81 |

Les plantes ont été transformées de manière stable avec un fragment de promoteur - 1024 fusionné au gène rapporteur GUS. Les résultats sont exprimés en pmol/h x mg de protéine et correspondent à la moyenne de 6 à 15 plantes de la descendance de chacune des 4 plantes F1 transformées indépendantes.

Exemple 4: Introduction de résistance herbicide dans le tabac:

Le fragment SacI-EcoRI de 3,5 kb issu du plasmide parental pRPA-RD-32(cf tableau 1) a été cloné dans les sites SacI-EcoRI de pBIN-19. La construction résultante, appelée pRPA-RD-53 comprend dans le cadre transcriptionnel les éléments suivants: l'élément de régulation *Tub* α *1* du maïs, le peptide de transit optimisé(OTP), le gène *aroA*, le terminateur *nos*.

Le plasmide parental pRPA-RD-53 a été transféré dans la souche d'*Agrobacterium tumefaciens* EHA-105(Hood *et al.* (1986) J.Bacteriol. 168, 1291) par croisement triparental et l'*Agrobacterium* résultant a été utilisé pour la transformation sur disque foliaire de tabac.

Les plantes de tabac régénérées, hautes d'environ 20 cm ont été pulvérisées en serre avec du glyphosate formulé sous forme de Round Up. Les plantes transformées contenant pRPA-RD-53, qui étaient viables et en bonne santé, ont montré une tolérance accrue au glyphosate.

SEQ. ID. NO. 1: Séquence du promoteur de *Tubα1* de maïs

CTCGAGAAGG ACTACGAGGA GGTTGGTGCT GAGTTTGATG AGGGTGAGGA AGGTGATGAT 60

GGTGATGAGT ACTAGAAGTA TCCTGATGCG GTCATCGTCA GGCTTGTGTG CTGCTCTTGT 120

CCCCGTTGTG GTTTGCAACA CCTGATGTTG TAAGACTTTC TGGTTATGTC CGCCCCGCTG 180

TGCCACTGGG TTATTAAGAA CGTCGTTATG GATGGTTGTC TACACTACAT TATTGCTTCT 240

CGATATTGGA AAACTGTTAT GCGCCTCGGT GGATTGTGTT GTTGTCGTAA TGTCATCACT 300

CATACGCCGC TGGGAATTTT GAGGCCTGTC AAGCATCAGG ATTGCGTTAT GAGTTAAATG 360

CTTCAGCGAC GTTTAAACTT GTCTAAGGTG CCATCTAGAT CATGAACTTG TCAAGGGTTG 420

CCACTTAGAT CATGAACTTC GTAAATATGT TTTTGGATCC AAAATATGTT TTTTATCCTT 480

AAGGGTGTGT TTGTGTGTTT GGTTGAATGT ATAAGAAGGG ATGAAAGAGG AATGTCATAA 540

TTTCTATAGT GTTTGGTTGA GAGACAAGTG AGGACGAGAT AAATACCTAA GAAGGGATGA 600

AAGAGGAATG CCACAATTTC TATAGTGTTT GGTTCAGAGA CAAGTGACAA TTTCTATAGT 660

GTTTGGTTGA GAGACAAGTG AGGGCGAGTA AATACCGCAA TAATTTTTTG GTGGCACCAA 720

ATTTTTGTGA AGTTGTATAC ATTTTGGACA CCAATAGAAA ATAGAATTAA AAAAATATAA 780

AACTGGTGTC ATTTAAATCA GTGTCACGTT ATTAAAATTT AAAACTATCA ACTAAAATTG 840

TCTAATGGAT TATTTATGTG GTTTTGTAAA GTTGTGGAGA TTAAACAACC AGTTTTGAAG 900

ATAAGTAAGT GAAATAGTCA AATAGACCGT ACTAAAGGTT AAGAATTTAG GTACACTTAC 960

GACTAGTTTA GATGCCGCAA AATGGGTTAA ATTTTTCTTC TTATTCAAAA TTAAATAATA 1020

AGGTGAATTT AACTACTCTA ATTTCCTCTG TTTTTTTAAC TCCCAAACTA TCCCTTATTC 1080

GTAATAATAG GAAGCGGTGA CAGTTTGGTG GTGAGAACTC AGGTATCAAC AAAAAGAAAT 1140

GTATTTTTGA AATATTTTGC TCGTAATGCC CTGCAAGGTT TCGATTTCCG TAGCCAGTAC 1200

ATGTCCGCTC TTGACCCAGG TACTGTGACA CGAACCAACC GACCGTTGAA CGGACGTGGA 1260

GCACGAACCA TTAAAACAAT CAAAATCTCA GGGGCTCAAA CGAAAAAACA CCGCCCCCTT 1320

CCCTCGCTTG CGCTGGCACT CCATCGTGGG CTCGTGGCCC AGGCTGTCGT TCTGTTCTAT 1380

AAAGCGAGAC GAGTGGGAGC AGGCGTAACC CTAATTGAGC ATCGCAGAGA TAGGCGTCTT 1440

CGTACTCGCC TACCTCCGCG GCTCAAACCT TTCCCCCTTC TCCCAATTCC TTCCGCCGGC 1500

CGCCGCTCCA CCCGTACGAC GACACCATG 1529

SEQ. ID. NO. 2 Séquence du promoteur de *Tubα2* de maïs

GAATTCCCTT TGTGAGAAAT CTCCACAAGT TGGAGCCTCT CACCCTTACA AGATTGATCA 60

CAATTAAACC ACAAGAGTAA GGGAGGGAAC AGAAACACAC ACAAGTGCTA GAGTCGCAGC 120

AATGACATGC ACACAAGTCA AGAAACGAGC ACACAACACA GCGCAACGAG GTCACAGTTC 180

```
AAACAAGTGC TCAAATCTTA AACACAATGA ATCGAATGCG TGCTTGCGGA GTCTAGACGT      240
TTTTTCAATG GAGGCTTGGT GTACTGCTCC ATGTGTCTAG GGGTCCCTTT TATAGCCCCA      300
AGGCAGCTAG GAGCCGTTGG AGCTCCATTT GGAAGGCCAT TGTTGCCTTC TATCCGTGGG      360
TGCACCGGAC AGTACGCGCT CGGGACGCGG CACATAATCC CATGATTGGC CGGTTTCCGC      420
TTCTGGGGGC ACCAGACGGT CCAGACGACC AGTGCGCCTG TCGACCGTTG GCCAGCGCTG      480
ACGTGGCCAC TAGTCGTTGC GTGGCTGATA CAACAGACTG TTCGGCGCAT TGCGGACCAT      540
CCGGTGAATT ATAGCTGATG TAGGCTAAAA AACCCGAGAG CAGCGAGTTC GGCCGAACCG      600
TGCACCAGAC TGTATGGTGG GTGGCATCAG ACCGTTCGGT GCTACACAGT CTAGCAACTT      660
TTCCCTGTTT CTTCTTTTGT CTTCTTTGTT TCTTTTGGAC TTCACTTAGC TGGGTCCCCT      720
GGCACTTAGA CAAATATGAT TAACACTCAA ATCAATTGAC TTAGTGTCTA GAGCATACCT      780
TTTAGCTTGA TCCATATAGC TTTGTACTAA GTCCTCTTCC GAGCTCATTT TGCCTCACAC      840
TTTTGCTTAA CATCATGTTA GTTCAAACAT CATGTGTTGT GCATCTAATC ACCAAACCAA      900
TATAGAAATG CCCAAGGACA CATTTCCCTT TCAGTCGGGG GGAGGGGGT TGGTGGTCGA       960
CGCCCCGGTA CGAAGTGGGT GGGGGCAGGC GAGAGGGGGT GCACCATGGG CCACCCAGTG     1020
CGTGGTCCGG TTTTGATCCA TGTAACTCTA TATAAATCTC TATTTAATTC GGTATAAAAT     1080
AGTTAAGATG AAAGAGAGAA TAAAATTTAG TAGATTTGAC AGTCATATAA AATTTCTAGA     1140
TCGACCCCTG TGGTGGGTGC GTCGAAATTT CTAGACCGCC CTAGGCCGGG GATGACACAT     1200
GGAACCGTGT ATGCACAAAG CTGCTGCATT ATAATTGTAG AGATTAATTA TGTTATTTAG     1260
GAAATAAAAG TTTAGGAATA GTATATAAAA CAAGGATTGA GCTCCAGATA TATAATAGGC     1320
CGAGTCCTGT AATTTTGTGA CATTTTTTTG AAACCAGGAT TGAGCTGCAG TTTTTAGTGT     1380
TAGAGTCCAG CGTCTCACAG GAGTGGTCCA ATTCAAATTC GAAAATGTAT CACCGCTGAA     1440
GCGAAAAATA TCATAAATTC ATAACGAACC AACCGACCGT TGCACGGACG AGAACTCGAC     1500
GAGACCGAAC CGTGAAAACA ACCGAAAGCA CAGGGGCTCA AACGAAACAA TCCCGCCCAC     1560
ACTTCCTTCG CCGGCTCATG GTGGCCACTG GCCAGGCTGT CATCCTGTTC TATAAAGCGA     1620
GCCGAGGGGA AGAGCCGGAA CCCTAGCCCA GCACCGCAGA GGCGCAGAGA CAGGCGTCTT     1680
CGTACTCGCC TATCTCCGCG ACTCAAAGCT TCTTCCATTT CCTACCGCCG CCGCTGCAGC     1740
TCCACCCCAT TCCGTCGACA CCATG                                           1765
```

SEQ. ID. NO. 3: Séquence du promoteur de *Tubα3* de maïs

```
AGATCTTGAT TCTGTGCAGT GCTGGTGATG GGAAAAAGCG AAAAACCATC GGTATGTTTT       60
TGACAAATAT GAAAATGGGA CAAAAACAAC ATGTGTGTTT TTTCGACCGT TTCCGCTTTT      120
CTTGTTTTAG TCACAATAGC TCGTTTTTAT CCACATATGA TATCTCATTT TAGATAATAC      180
```

```
ATGAACAAAT CATAATTGAT TATATCATAT CTCAACAAAT TAACCCGTAA TGAATTATTT    240
TTCTTTGATA GTCATATGTA CATTACAATA TTTCGCTTCC ATATGTATGG ATGTGATGTT    300
TTAATCGATT GCAACACTAC TTTTATTTTT ATACTCTATG TGACAATTAT TTCCGCTTTT    360
ATTTACATCT TATTCCGATC TGTTATCGAT ATCGATTTGT TCCGTCCCGT TTTTATCTTA    420
TTTCTGATAG TTCCAATTTA ATCTTATTTT CGAAATAAAG TATGAAAATA AAAATAAGAG    480
AGATTGTTAC GTTCGATCCG GTTTTGAACC CTAGCTATAC TTGCCCGTTG TTGCAACTGG    540
CCGGCCATTC CATAGGCGGG CACAGTCAGC ACTCAGCAGT GACAGAGTGC GCGTGCGACA    600
CACAGTTTCA AATTTCAAAA CTGAAACGGG CGGCTATAAA CAGAACCCGC TGCTCCCAGG    660
AGCCTCACGC AGATAAATTC ACCCACATCA ATGGGGCCCA AATATTTATA ACCATCTATT    720
GGTCCCACAT GTTCGTGTCA CAACATCCTC TACCGCAGGT AAAGATAGCC GTCTCGCCAA    780
GACCCCGAGC CCGCCGCTGC CCGGACCCGC CGCCAGCTCA CACCCACCGT TGCCGGCCGC    840
TGAGCCGTTC GAAGCCAAAA CGGTCGTTAA CCACCCAGCT GCCCGTCGGC TACCATCACG    900
CCGTTAGCCC CGAACCAGAC GGCGGCTAGG TCTTCCGCCG CGCGCCGCGC CATCACGGGC    960
CGGCCGCGGC CTTCTTTCCC ACGCTGCCTA TAAAAGCCGC CGCGGGGCTG AGCAGCATTA   1020
TCGCTTCAGC TCGGCGTCTT CACAAACGCC GGCGCAAACT CTCGCCCGAG CCCGACAGAT   1080
CTTCAATTCC CCATTCCGCC CACCGATCGA CCTTCACGCC AGTCTCGGTC TCTTCCGAAG   1140
GCGTCGCGCG CGGTTGTTTG AGAGGGGAGG AGGAAGATG                          1179
```

SEQ. ID. NO. 4: Peptide de transit modifié

```
GAATTCCGAA AGACAAAGAT TATCGCCATG GCTTCGATCT CCTCCTCAGT CGCGACCGTT     60
AGCCGGACCG CCCCTGCTCA GGCCAACATG GTGGCTCCGT TCACCGGCCT TAAGTCCAAC    120
GCCGCCTTCC CCACCACCAA GAAGGCTAAC GACTTCTCCA CCCTTCCCAG CAACGGTGGT    180
GGAAGAGTTC AATGTATGCA GGTGTGGCCG GCCTACGGCA ACAAGAAGTT CGAGACGCTG    240
TCGTACCTGC CGCCGCTGTC AATGGCGCCC ACCGTGATGA TGGCCTCGTC GGCCACCGCC    300
GTCGCTCCGT TCCAGGGGCT CAAGTCCACC GCCAGCCTCC CCGTCGCCCG CCGCTCCTCC    360
AGAAGCCTCG GCAACGTCAG CAACGGCGGA AGGATCCGGT GCATG                    405
```

## REFERENCES

**Amstrong, C.L, Petersen, W.L., Buchholz, W.G. and Bowen, B.A.** (1990) Factors affecting PEG-mediated stable transformation of maize protoplasts. *Plant Cell Reports*, **9**, 335-339.

**Bedinger, P. and Edgerton, M.D.** (1990) Developmental staging of maize microspores reveals a transition in developing microspore proteins. *Plant Physiol.* **92**, 474-479.

**Carpenter, J.L., Ploense, S.E., Snustad, D.P. and Silflow, C.D.** (1992) Preferential Expression of an $\alpha$-Tubulin Gene of Arabidopsis in Pollen. *Plant Cell*, **4**, 557-571.

**Chu, C., Wang, C., Sun, C., Hsu, C., Yin, K. and Chu, C.** (1975) Establishment of an efficient medium for anther culture of rice through comparative experiments on the nitrogen sources. *Scientia Sinica*, **18**, 659-668.

**Feldman, L.J.** (1984) Regulation of root development. *Ann. Rev. Plant Physiol.* **35**, 223-242.

**Gorman, C.M., Moffat, L.F. and Howard, B.H.** (1982) *Mol. Cell. Biol.* **2**, 1044-1051.

**Jefferson, R.A., Kavanagh, T.A. and Bevan, M.W.** (1987a) GUS fusions: β-glucuronidase as a sensitive and versatile gene fusion marker in higher plants. *EMBO J.* **6**, 3901-3907.

**Jefferson, R.A.** (1987b) Assaying Chimeric Genes in Plants: The GUS gene fusion system. *Plant Mol. Biol. Rep.* **5**, 387-405.

**Kopczak, S.D., Haas, N.A., Hussey, P.J., Silflow, C.D. and Snustad, D.P.** (1992) The Small genome of Arabidopsis Contains at Least Six Expressed α-Tubulin Genes. *Plant Cell*, **4**, 539-547.

**Kosugi, S., Ohashi, Y., Nakajima, K. and Arai, Y.** (1990) An improved assay for β-glucuronidase in transformed cells: Methanol almost completely suppresses a putative endogenous β-glucuronidase activity. *Plant Sci.* **70**, 133-140.

**Mandaron, P., Niogret, M.F., Mache, R and Moneger, F.** (1990) In vitro protein synthesis in isolated microspores of *Zea mays* at several stages of development. *Theor. Appl. Genet.* **80**, 134-138.

**Mc.Cormick, S.**, (1991) Molecular analysis of male gametogenesis in plants. *Trends Genet.* **7**, 289-303.

**Montoliu, L., Puigdomènech, P. and Rigau, J.** (1990) The *Tubα3 gene* from *Z.mays*: structure and expression in dividing plant tissues. *Gene*, **94**, 201-207.

**Montoliu, L.,Rigau. J. and Puigdomènech, P.** (1989) A tandem of α-tubulin genes preferentially expressed in radicular tissues of *Z.mays. Plant Biol.* **14**, 1-15.

**Montoliu, L., Rigau, J. and Puigdomènech, P.** (1992) Analysis by PCR of the number of homologous genomic sequences to α-tubulin in maize. *Plant Sci.* **84**, 179-185.

**Murashigue, T. and Skoog. F.** (1962). A revised medium for rapid growth and bioassays with tobacco tissue cultures. *Physiol. Plant*, **15**, 473-497.

**Paszkowski, J. and Saul, M.W.** (1986) Direct gene transfer to plants. In *Methodsin Enzymology*. Vol. 118. *Plant Molecular Biology*. Academic Press Inc. pp. 669-684.

**Snustad, D.P., Haas, N.A., Kopczak, S.D. and Silflow, C.D.** (1992) The Small genome of Arabidopsis Contains at Nine Expressed β-Tubulin Genes. *Plant Cell*, **4**, 549-556.

**Stinson, J.R., Eisenberg,A.R., Willin, R.P., Pe, M.E., Hanson, D.D. and Mascarenhas, J.P.** (1987) Genes expressed in the male gametophyte of flowering plants and their isolation. *Plant Physiol.* **83**, 442-447.

**Villemur, R., Joyce, C.M., Haas, N.A., Godbard, R.H., Kopczak, S.D., Hussey, P.J., Snustad, D.P. and Silflow, C.D.** (1992) alpha-Tubulin Gene Family of Maize (Zea-Mays L). Evidence for 2 Ancient alpha-Tubulin Genes in Plants. *J. Mol. Biol.* **227**, 81-96.

## Revendications

**1.** Acide nucléique isolé issu d'un gène de tubuline α de maïs comprenant au moins un élément de régulation, qui commande au moins une expression spécifique dans le pollen, les racines, les zones méristématiques et les embryons immatures.

**2.** Acide nucléique isolé selon la revendication 1, caractérisé en ce que le gène est le gène *Tub* α *1* de maïs.

**3.** Acide nucléique isolé selon la revendication 1, caractérisé en ce que le gène est le gène *Tub* α *2* de maïs.

**4.** Acide nucléique isolé selon la revendication 1, caractérisé en ce que le gène est le gène *Tub* α *3* de maïs.

**5.** Acide nucléique isolé selon l'une des revendications 1 à 4, caractérisé en ce que l'élément de régulation commande une expression d'un gène sous son contrôle détectable dans le pollen.

**6.** Acide nucléique isolé selon l'une des revendications 1 à 4, caractérisé en ce que l'élément de régulation commande une expression d'un gène sous son contrôle détectable dans les racines.

**7.** Acide nucléique isolé selon l'une des revendications 1 à 4, caractérisé en ce que l'élément de régulation commande une expression d'un gène sous son contrôle détectable dans les méristèmes.

**8.** Acide nucléique isolé selon l'une des revendications 1 à 4, caractérisé en ce que l'élément de régulation commande une expression d'un gène sous son contrôle détectable dans les embryons immatures.

**9.** Acide nucléique selon la revendication 5, caractérisé en ce que l'élément de régulation comprend les nucléotides 1 à 1348 de SEQ ID N°: 1.

**10.** Acide nucléique selon la revendication 9, caractérisé en ce que l'élément de régulation comprend les nucléotides 1295 à 1348 de SEQ ID N°: 1.

**11.** Acide nucléique selon la revendication 6, caractérisé en ce que l'élément de régulation comprend les nucléotides 1 à 1529 de SEQ ID N°: 1.

**12.** Acide nucléique selon la revendication 11, caractérisé en ce que l'élément de régulation comprend les nucléotides 1 à 1115 de SEQ ID N°: 1.

**13.** Acide nucléique selon l'une des revendications 7 et 8, caractérisé en ce que l'élément de régulation comprend les nucléotides 1 à 695 de SEQ ID N°: 3.

**14.** Acide nucléique selon la revendication 13, caractérisé en ce que l'élément de régulation comprend les nucléotides 542 à 695 de SEQ ID N°: 3.

**15.** Acide nucléique selon l'une des revendications 7 et 8, caractérisé en ce que l'élément de régulation comprend les nucléotides 1 à 1076 de SEQ ID N°: 3.

**16.** Gène chimère de plante comprenant une partie suffisante d'un ADN promoteur selon l'une des revendications 1 à 15 capable de fonctionner dans les plantes et fonctionnellement relié à la séquence codante pour pouvoir faire exprimer le gène hétérologue.

**17.** Gène chimère de plante selon la revendication 16 caractérisé en ce qu'il comprend, entre le promoteur et la séquence codante, un autre élément de régulation du gène.

**18** Gène chimère de plante selon la revendication 17 caractérisé en ce que l'élément de régulation du gène est un promoteur qui fonctionne dans les plantes.

**19.** Gène chimère de plante selon la revendication 18, caractérisé en ce que l'élément de régulation additionnel est le promoteur CaMV 35S du virus de la mosaïque du choux-fleur.

**20.** Gène chimère de plante selon la revendication 18, caractérisé en ce que l'élément de régulation additionnel est un promoteur histone de plante.

**21.** Gène chimère de plante selon la revendication 18, caractérisé en ce que l'élément de régulation additionnel est un promoteur de gène actine de riz.

**22.** Gène chimère de plante selon l'une des revendications 16 à 21, caractérisé en ce qu'il comprend un gène hétérologue compris dans le groupe comprenant le gène codant pour une enzyme du métabolisme des lipides, une désaturase, un gène de résistance herbicide ou un gène codant pour la 5' énolpyruvyl shikimate-3-phosphate synthase, l'acétolactase et la 3-hydroxyphényl pyruvate dioxygenase(HPPO).

**23.** Gène chimère de plante selon la revendication 22, caractérisé en ce que le gène hétérologue est le gène aroA de résistance au glyphosate.

**24.** Vecteur de transformation des plantes caractérisé en ce qu'il comprend un gène chimère de plante selon l'une des revendications 16 à 23.

**25.** Cellule végétale comprenant un vecteur selon la revendication 24.

**26.** Plante ou descendance de plante, caractérisée en ce qu'elle a été régénérée à partir d'une cellule selon la revendication 25.

**27.** Plante selon la revendication 26, caractérisée en ce qu'elle est une monocotylédone.

**28.** Plante selon la revendication 27, caractérisée en ce qu'elle est du maïs ou une céréale.

**29.** Plante selon la revendication 26, caractérisée en ce qu'elle est une dicotylédone.

**30.** Plante selon la revendication 29, caractérisée en ce qu'elle est le tabac, le coton ou le soja.

**31.** Procédé d'obtention d'une plante avec une propriété agronomique améliorée, caractérisé en ce qu'il comprend successivement:

a) la transformation d'une cellule végétale avec un vecteur selon la revendication 24, et

b) la régénération de la plante.

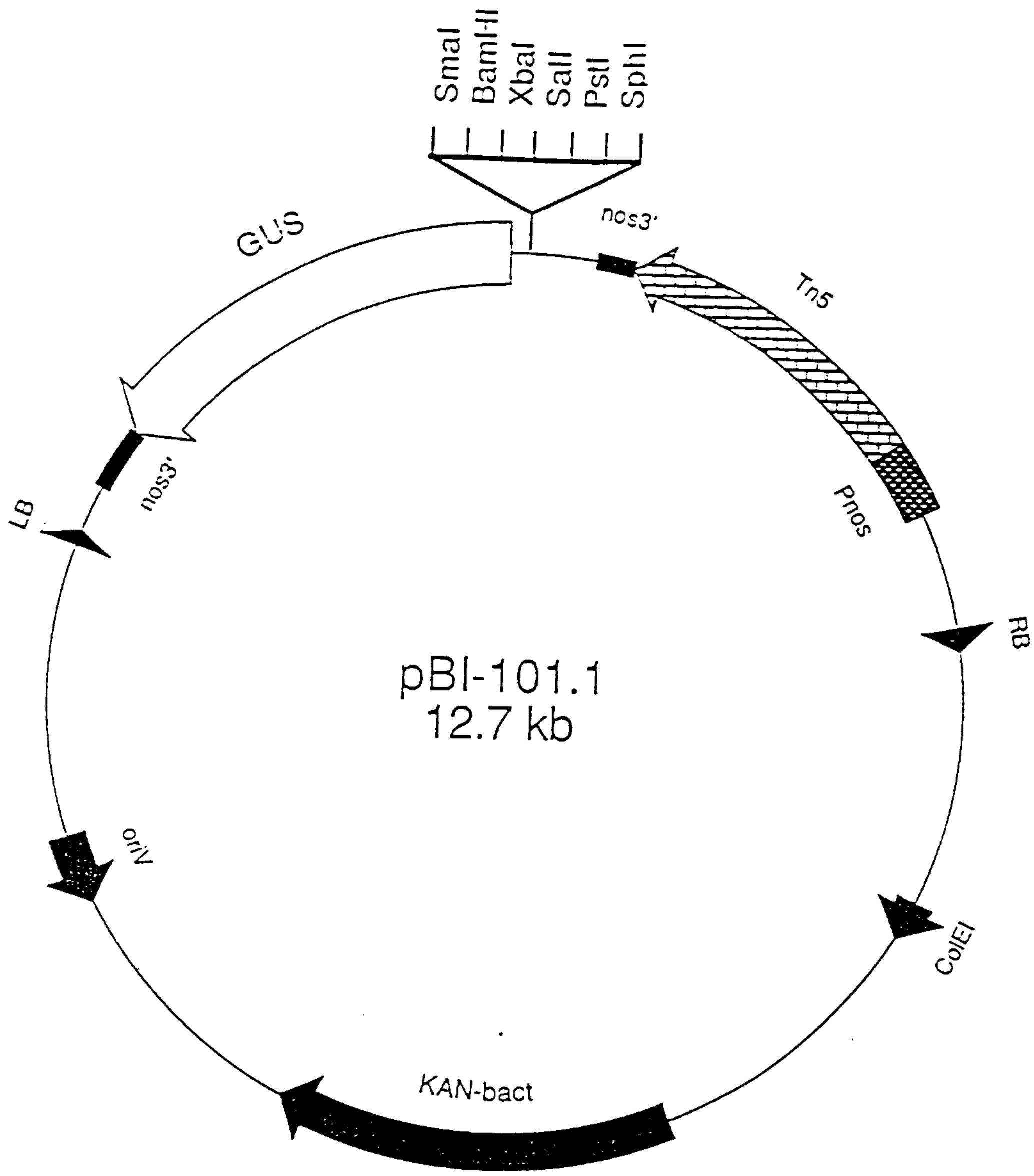
SmaI
BamHI
XbaI
SalI
PstI
SphI
GUS
nos3'
Tn5
Pnos
RB
LB
nos3'
pBI-101.1
12.7 kb
oriV
ColEI
KAN-bact

Fig. 1

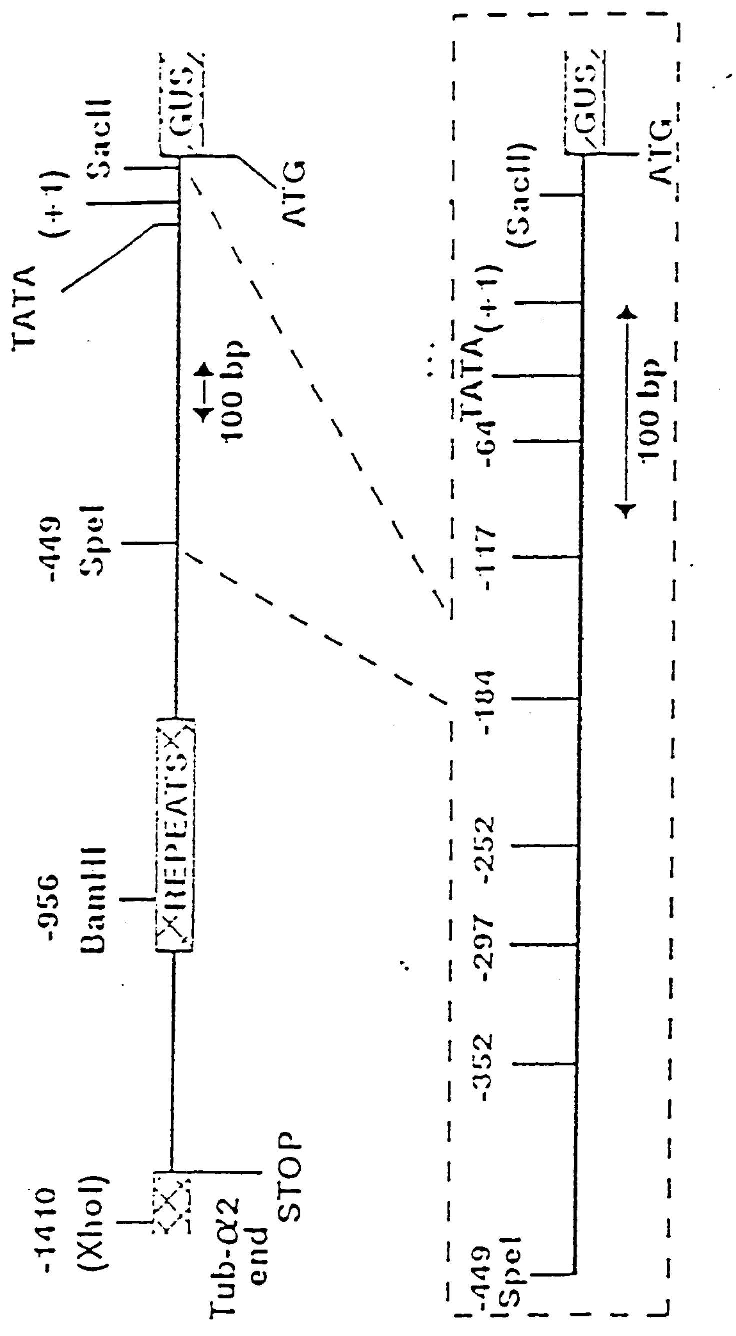
-1410
(XhoI)
Tub-α2
end
STOP
-956
BamHI
REPEATS
-449
SpeI
100 bp
TATA
(+1)
SacII
GUS
ATG
-449
SpeI
-352
-297
-252
-184
-117
-64
TATA
(+1)
(SacII)
GUS
ATG
100 bp

Fig. 2

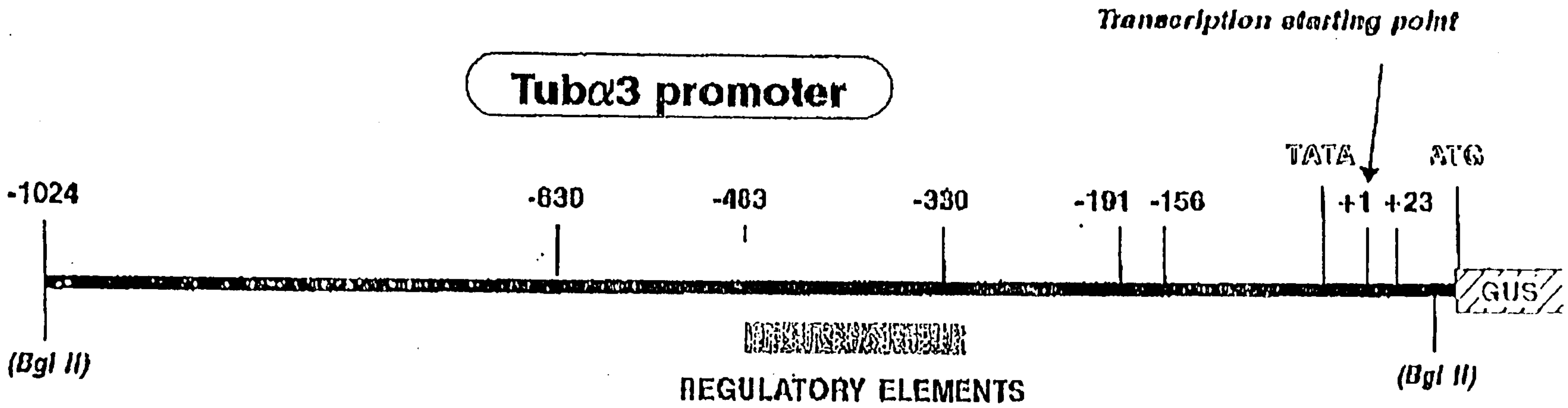
Tubα3 promoter
Transcription starting point
TATA
ATG
-1024
-630
-403
-330
-101
-156
+1
+23
GUS
(Bgl II)
(Bgl II)
REGULATORY ELEMENTS

Fig. 3

Office européen des brevets

# RAPPORT DE RECHERCHE EUROPEENNE

Numero de la demande
EP 94 42 0306

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| P,X | THE PLANT JOURNAL, vol.4, no.6, Décembre 1993 pages 1043 - 1060 RIGAU, J., ET AL. 'Analysis of a maize alpha-tubulin gene promoter by transient gene expression and transgenic tobacco plants' * le document en entier * | 1-31 | C12N15/82 C12N15/29 C12N5/10 A01H5/00 |
| X | PLANT MOLECULAR BIOLOGY, vol.14, 1989 pages 1 - 15 MONTOLIU, L., ET AL. 'A tandem of alpha-tubulin genes preferentially expressed in radicular tissues from Zea mays' | 1-9,11, 12 | |
| Y | * le document en entier * | 16,22-31 | |
| Y | THE PLANT CELL, vol.4, no.5, Mai 1992 pages 557 - 571 CARPENTER, J.L., ET AL. 'Preferential expression of an alpha-tubulin gene of Arabidopsis in pollen' * le document en entier * | 16,22-31 | DOMAINES TECHNIQUES RECHERCHES (Int.Cl.6) C12N A01H |
| X | GENE, vol.94, 1990 pages 201 - 207 MONTOLIU, L., ET AL. 'The tubalpha3 gene from Zea mays: structure and expression in dividing plant tissues' * le document en entier * | 1-8,13, 15 | |
| Y | EP-A-0 507 698 (RHONE-POULENC) 7 Octobre 1992 * le document en entier * | 22,23 | |

-/--

Le présent rapport a été établi pour toutes les revendications

| Lieu de la recherche | Date d'achèvement de la recherche | Examinateur |
|---|---|---|
| LA HAYE | 1 Février 1995 | Maddox, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 94 42 0306

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | PLANT MOLECULAR BIOLOGY, vol.21, Mars 1993 pages 937 - 942 CARPENTER, J.L., ET AL. 'Semi-constitutive expression of an Arabidopsis thaliana alpha-tubulin gene' * le document en entier * ----- | 1-31 | |
| | | | DOMAINES TECHNIQUES RECHERCHES (Int.Cl.6) |

Le présent rapport a été établi pour toutes les revendications

| Lieu de la recherche | Date d'achèvement de la recherche | Examinateur |
|---|---|---|
| LA HAYE | 1 Février 1995 | Maddox, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)